Europäisches Patentamt

European Patent Office

Office européen des brevets

(11) Veröffentlichungsnummer: **0 195 207**
**A2**

## (12) EUROPÄISCHE PATENTANMELDUNG

(21) Anmeldenummer: **86101228.4**

(22) Anmeldetag: **30.01.86**

(51) Int. Cl.⁴: **G 01 K 13/00**
**G 01 K 1/02**

(30) Priorität: **16.03.85 DE 3509503**

(43) Veröffentlichungstag der Anmeldung:
**24.09.86 Patentblatt 86/39**

(84) Benannte Vertragsstaaten:
**AT BE CH DE FR GB IT LI LU NL SE**

(71) Anmelder: **Frohn, Hermann-Josef, Dr.**
**Am Römerlager 4**
**D-5300 Bonn 1(DE)**

(72) Erfinder: **Frohn, Hermann-Josef, Dr.**
**Am Römerlager 4**
**D-5300 Bonn 1(DE)**

(74) Vertreter: **Selting, Günther, Dipl.-Ing. et al,**
**Deichmannhaus am Hauptbahnhof**
**D-5000 Köln 1(DE)**

(54) **Vorrichtung zur Empfängnisregulierung.**

(57) Die Vorrichtung weist ein in die Gebärmutter einsetzbares Gehäuse (11) auf, das eine Temperaturmeßeinrichtung (16) enthält, deren Temperaturwerte in bestimmten Zeitintervallen in einen Datenspeicher (17) eingegeben werden. Eine Sende- und Empfangseinrichtung (19) sendet auf ein externes Befehlssignal hin sämtliche gespeicherten Temperaturwerte aus. Auf diese Weise kann über einen Monat hinweg die Körpertemperatur sehr genau und in konstanten Intervallen gemessen werden, um den Zeitpunkt des Eisprungs festzustellen. In Abhängigkeit davon kann gezielt eine Befruchtung durchgeführt werden oder unterbleiben.

FIG.2

Vorrichtung zur Empfängnisregulierung

Die Erfindung betrifft eine Vorrichtung zur Empfängnisregulierung, mit einer Temperaturmeßeinrichtung zur Messung der Körpertemperatur.

Es ist bekannt, daß die Körpertemperatur der Frau zur Zeit des Eisprungs abnimmt, um anschließend wieder anzusteigen. Zur Zeit des Eisprungs ist die Befruchtungsmöglichkeit gegeben. Je genauer man den Zeitpunkt des Eisprungs bestimmen kann, umso sicherer kann eine Befruchtung erreicht bzw. vermieden werden. Nach Knaus-Ogino soll die Körpertemperatur der Frau täglich mit einem Thermometer gemessen werden. Durch regelmäßige Messung und Aufzeichnung der Körpertemperatur kann dann eine Aussage über den zeitlichen Befruchtungsbereich erlangt werden. Das Messen mit einem Körperthermometer ist langwierig und zeitraubend und zudem ungenau. Außerdem werden die regelmäßigen Temperaturmessungen gelegentlich vergessen, so daß genaue und regelmäßige Aufzeichnungen der Körpertemperatur in der Praxis selten durchgeführt werden.

Der Erfindung liegt die Aufgabe zugrunde, eine Vorrichtung der eingangs genannten Art zu schaffen, mit der die Körpertemperatur mit hoher Genauigkeit und in regelmäßigen Zeitabständen gemessen wird, um eine zuverlässige Temperaturdatentabelle über einen längeren Zeitraum zu erhalten.

Zur Lösung dieser Aufgabe ist erfindungsgemäß vorgesehen, daß die Temperaturmeßeinrichtung in einem in den Körper einführbaren Gehäuse untergebracht ist, das außerdem einen Datenspeicher enthält, der unter Steuerung durch einen Zeitgeber in festgelegten Zeitintervallen die Temperaturwerte speichert, und daß eine Sende- und Empfangseinrichtung vorgesehen ist, die auf ein externes Befehlssignal hin Daten aus dem Datenspeicher aussendet.

Die erfindungsgemäße Vorrichtung wird in den Körper der Frau eingeführt, beispielsweise in die Gebärmutter. Die Temperaturmeßeinrichtung ist vollständig in den Körper eingebettet und nimmt daher die Körpertemperatur an. In bestimmten Zeitabständen, z.B. einmal täglich, wird der Temperaturwert in den elektronischen Speicher eingespeichert und dort festgehalten. Das Auslesen des elektronischen Speichers erfolgt auf das externe Befehlssignal hin. Der Speicher gibt dann die in ihm enthaltenen Temperaturwerte an ein externes Gerät aus, das diese Temperaturwerte ausdruckt oder auf andere Weise lesbar macht. Auf diese Weise erhält man ein zuverlässiges Protokoll über den zeitlichen Verlauf der Körpertemperatur und kann auf diese Weise den Zeitpunkt des Eisprungs ermitteln.

Die erfindungsgemäße Vorrichtung kann zur Empfängnisverhütung, aber auch zur gewollten Empfängnis, benutzt werden, je nach dem Verhalten der Frau in dem festgestellten Befruchtungzeitraum. In Abhängigkeit von der Speicherkapazität des Speichers kann die Temperaturmessung auch beispielsweise stündlich durchgeführt werden. Der Abruf der gemessenen und gespeicherten Daten erfolgt zweckmäßigerweise auf drahtlosem Wege. So kann beispielsweise der Arzt einmal im Monat über ein externes Gerät, das mit der Sende- und Empfangseinrichtung drahtlos zusammenarbeitet, die gespeicherten Daten abrufen und der Patientin entsprechende Empfehlungen geben. Die erfindungsgemäße Vorrichtung eignet sich nicht ausschließlich zur Empfängnisregulierung, sondern sie kann generell dazu benutzt werden, die Körpertemperatur über einen bestimmten Zeitraum zuverlässig zu kontrollieren, ohne daß der Patient selbst regelmäßige Temperaturmessungen vornehmen müßte.

Die erfindungsgemäße Vorrichtung kann in den Körper implantiert werden. Als Ort für die Implantation ist bei Frauen insbesondere die Gebärmutter geeignet. Es ist bereits bekannt, empfängnisverhütende Spiralen in die Gebärmutter einzusetzen. Die erfindungsgemäße Vorrichtung wird prinzipiell in ähnlicher Weise in der Gebärmutter befestigt wie die bekannten Spiralen.

Zur Befestigung des Gehäuses in der Gebärmutter weist das Gehäuse mindestens ein ausziehbares langgestrecktes Halteorgan zur Verankerung des Gehäuses an einem Eileiter auf. Das Gehäuse wird durch ein Rohr hindurch in die Gebärmutter eingeführt, so daß jedes von zwei Halteorganen in einen der Eileiter eintritt und sich an der Eileiterwand abstützt. Auf diese Weise wird das Gehäuse in der Gebärmutter festgehalten. Das Gehäuse kann einen Zugfaden aufweisen, der das spätere Herausziehen der Vorrichtung aus dem Körper der Patientin
erleichtert.

Das Einführen der Vorrichtung in den Körper einer Patientin ist mit einem entsprechenden Rohr relativ einfach
durchführbar. Beim Herausnehmen des Gehäuses aus dem
Körper muß aber darauf geachtet werden, daß das bzw.
die Halteorgane die Gebärmutterwand nicht beschädigen.
Außerdem besteht die Gefahr, daß ein befruchtetes Ei,
das sich in der Gebärmutter befindet, durch ein Halteorgan beschädigt wird. Um dies zu vermeiden, ist gemäß
einer bevorzugten Weiterbildung der Erfindung vorgesehen, daß das Halteorgan durch eine Feder in Einzugsrichtung gespannt ist und daß eine durch ein zweites
externes Befehlssignal auslösbare Blockiereinrichtung
vorgesehen ist, die im Auslösefall die Feder freigibt,
um das Halteorgan in das Gehäuse einzuziehen. Vor dem
Entfernen des Gehäuses aus dem Körper werden die Halteorgane in das Gehäuse eingezogen. Das Gehäuse ist vorzugsweise rund, d.h. kugelförmig oder eiförmig ausgebildet und enthält Platz zur Aufnahme der eingezogenen
Halteorgane. Nach dem Einziehen der Halteorgane kann
das Gehäuse problemlos aus dem Körper herausgezogen
werden. Zweckmäßigerweise besteht jedes Halteorgan aus
einem flexiblen Teleskopstab.

Im folgenden wird unter Bezugnahme auf die Zeichnungen
ein Ausführungsbeispiel der Erfindung näher erläutert.

Es zeigen:

Fig. 1 eine schematische Darstellung der Gebärmutter
mit eingesetzter Vorrichtung,
Fig. 2 eine schematische Schnittdarstellung des Ge-

0195207

häuses der Vorrichtung, und

Fig. 3 ein elektrisches Blockschaltbild.

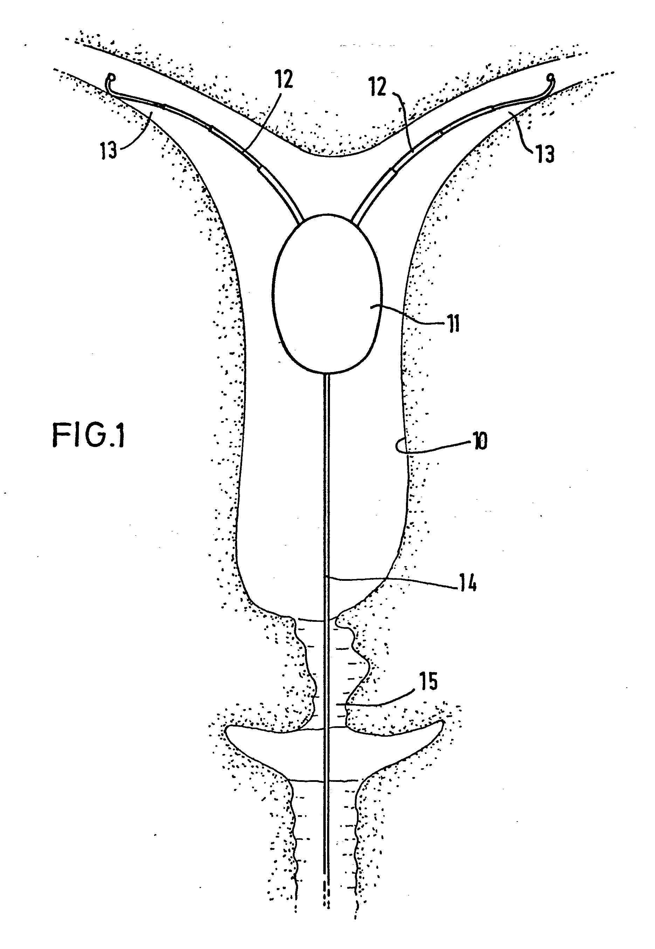

Gemäß Figur 1 ist in die Gebärmutter 10 ein Gehäuse 11 eingesetzt, von dem zwei langgestreckte Stützorgane 12 abstehen, die in die Eileiter 13 hineinragen und die Verankerung des Gehäuses 11 im Innern der Gebärmutter bewirken. Von dem den Halteorganen 12 abgewandten Ende des Gehäuses 11 hängen Fäden 14 herab, die durch den Muttermund 15 hindurchführen. Diese Fäden dienen zum späteren Herausziehen des Gehäuses 11 aus dem Körper.

Das Gehäuse 11 ist kugelförmig oder eiförmig ausgebildet. Es besteht aus einem rotationssymmetrischen Körper, der keinerlei Ecken aufweist. Das Material des Gehäuses ist körperverträglich.

In dem Gehäuse 11 befinden sich eine elektronische Temperaturmeßeinrichtung 16, ein Datenspeicher 17, ein Zeitgeber 18 sowie eine Sende- und Empfangseinrichtung 19. Ferner ist in dem Gehäuse 11 eine Batterie 20 zur Versorgung der genannten Komponenten enthalten. Sämtliche Komponenten sind miniaturisiert, so daß das Gehäuse 11 so klein wie möglich ausgeführt werden kann.

Die Temperaturmeßeinrichtung 16 erzeugt ständig eine digitale Signalkombination, die die herrschende Körpertemperatur repräsentiert. Diese Signalkombination wird von dem Datenspeicher 17 jeweils dann übernommen, wenn der Zeitgeber 18 ein entsprechendes Steuersignal liefert. Dies geschieht beispielsweise in jeder Stunde oder an jedem Tag einmal. Es ist auch möglich, die Intervalle, in denen die Temperaturwerte in den Speicher 17 übernommen werden, durch externe Befehlssignale zu verändern. Im Datenspeicher 17 werden die Temperaturwerte und die zugehörigen Zeitwerte in digitaler Form gespeichert. Die Sende- und Empfangseinrichtung 19 ist mit einer Empfangsantenne 20 und einer Sendeantenne 21 ausgestattet, die hier aus Gründen der Übersichtlichkeit einzeln dargestellt sind, jedoch auch aus einer einzigen Antenne bestehen können. Die Empfangsantenne 20 empfängt ein von einem externen Gerät ausgesandtes Befehlssignal zum Abruf der Daten aus dem Datenspeicher 17. Der Datenspeicher 17 liefert daraufhin die in ihm enthaltenen Temperatur- und Zeitdaten an die Sende- und Empfangseinrichtung 19, die diese Daten über die Sendeantenne 21 abstrahlt. Die Daten werden von dem externen Gerät zwischengespeichert, um nachfolgend ausgedruckt oder auf andere Weise sichtbar gemacht zu werden.

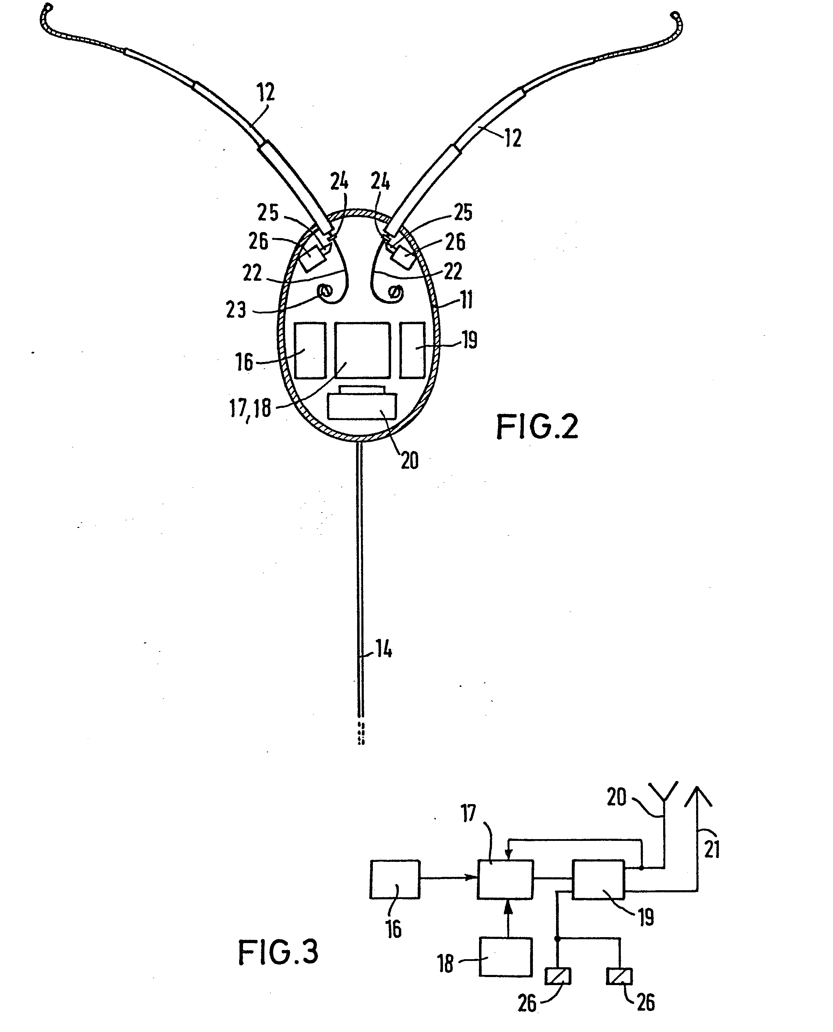

Die Halteorgane 12, die aus dem Gehäuse 11 herausragen, sind als Teleskopstäbe ausgebildet, die in den Figuren 1 und 2 im ausgestreckten Zustand dargestellt sind. Die Teleskopstäbe bestehen aus einem elastischen Material. Durch jedes Halteorgan 12 erstreckt sich eine Feder 22, die im vorliegenden Fall als Spiralfeder ausgebildet ist. Das Ende der Feder 22 ist an einem Befestigungspunkt 23 des Gehäuses 11 fixiert. Jede der Federn 22 hat das Bestreben, sich um den Befestigungspunkt 23 herum spiralförmig aufzuwickeln. An der Feder 22 ist ein Vorsprung 24 angebracht, an dem ein Blockierelement 25 angreift. Das Blockierelement 25 wird von einem Elektromagneten 26, der in dem Gehäuse 11 untergebracht ist, gesteuert. Es ist von einer (nicht dargestellten) Feder in Richtung auf die Blockierstellung vorgespannt, in der es an dem Ansatz 24 angreift und das Einziehen der Feder 22 in das Gehäuse 11 verhindert. Wenn durch einen Stromimpuls durch den Elektromagneten 26 das Blockierelement 25 zurückgezogen wird, wickelt sich die Feder in dem Gehäuse 11 um den Festpunkt 23 herum auf, wodurch die einzelnen Abschnitte des Teleskopstabes 12 ineinandergeschoben und in das Gehäuse 11 eingezogen werden. Danach kann das Gehäuse 11 durch Ziehen an den Fäden 14 ohne die Gefahr von Beschädigungen der Gebärmutter aus dem Körper herausgezogen werden. Die Betätigung der Elektromagnete 26 zum Einziehen der Teleskopstangen 12 erfolgt durch die Sende- und Empfangseinrichtung 19, wenn diese ein entsprechendes zweites externes Befehlssignal empfängt.

ANSPRÜCHE

1. Vorrichtung zur Empfängnisregulierung, mit einer Temperaturmeßeinrichtung zur Messung der Körpertemperatur,
dadurch gekennzeichnet, daß
die Temperaturmeßeinrichtung (16) in einem in den Körper einführbaren Gehäuse (11) untergebracht ist, das außerdem einen Datenspeicher (17) enthält, der unter Steuerung durch einen Zeitgeber (18) in festgelegten Zeitintervallen die Temperaturwerte speichert und daß eine Sende- und Empfangseinrichtung (19) vorgesehen ist, die auf ein externes Befehlssignal hin Daten aus dem Datenspeicher (17) aussendet.

2. Vorrichtung nach Anspruch 1,
dadurch gekennzeichnet, daß
das Gehäuse (11) mindestens ein ausziehbares langgestrecktes Halteorgan (12) zur Verankerung des Gehäuses an einem Eileiter aufweist.

3. Vorrichtung nach Anspruch 1 oder 2,
dadurch gekennzeichnet, daß
das Gehäuse (11) mindestens einen Zugfaden (14) aufweist.

4. Vorrichtung nach einem der Ansprüche 1 bis 3,
dadurch gekennzeichnet, daß
das Halteorgan (12) durch eine Feder (22) in Einzugsrichtung gespannt ist und daß eine durch ein zweites externes Befehlssignal auslösbare Blockiervorrichtung (25,26) vorgesehen ist, die im Auslösefall die Feder (22) freigibt, um das Halteorgan (12) in das Gehäuse (11) einzuziehen.

0195207

5. Vorrichtung nach einem der Ansprüche 1 bis 4, dadurch gekennzeichnet, daß das Halteorgan (12) aus einem flexiblen Teleskopstab besteht.

FIG.1

FIG.2

FIG.3